# EUROPÄISCHE PATENTANMELDUNG

(11) **EP 1 970 435 A1**
(43) Veröffentlichungstag der Anmeldung: **17.09.2008**
(21) Anmeldenummer: 08450033.9
(22) Anmeldetag: 12.03.2008
(51) Int. Cl.: C12M 1/107, C12M 1/113

(54) **Vorrichtung zur Gärgutbeschickung einer Gäranlage**

(30) Priorität: 13.03.2007 AT 3992007
(71) Anmelder: MARATON Energie Technik GmbH, 5211 Friedburg (AT)
(72) Erfinder: Metzger, Reinhard, 5310 Mondsee (AT)
(74) Vertreter: Hübscher, Helmut

(57) **Zusammenfassung**

Es wird eine Vorrichtung zur Gärgutbeschickung einer Gäranlage mit einer das Gärgut aufnehmenden Vorratskammer (1) und mit einem in der Vorratskammer (1) vorgesehenen Schubboden (3) beschrieben, der eine in einer stirnseitigen Austragsöffnung (4) der Vorratskammer (1) mündende Förderfläche (5) für das Gärgut bildet. Um vorteilhafte Beschickungsbedingungen zu schaffen, wird vorgeschlagen, dass an die stirnseitige Austragsöffnung (4) der Vorratskammer (1) ein gegen die Austragsöffnung (4) umfangsseitig offener Mischbehälter (13) angeschlossen ist, der wenigstens eine von seinem Boden (14) aufragende, mit Zerkleinerungswerkzeugen (16) versehene Mischerschnecke (15) besitzt, und dass der Boden (14) des Mischbehälters (13) in einem Höhenabstand unterhalb der Förderfläche (5) des Schubbodens (3) liegt.

## Beschreibung

Die Erfindung bezieht sich auf eine Vorrichtung zur Gärgutbeschickung einer Gäranlage mit einer das Gärgut aufnehmenden Vorratskammer und mit einem in der Vorratskammer vorgesehenen Schubboden, der eine in einer stirnseitigen Austragsöffnung der Vorratskammer mündende Förderfläche für das Gärgut bildet.

Zur Beschickung von Gäranlagen für eine Biogaserzeugung wird das jeweils benötigte Gärgut, üblicherweise nachwachsende Rohstoffe, schubweise einer Förderschnecke aufgegeben, die die Gäranlage mit dem Gärgut beschickt. Das Gärgut wird zu diesem Zweck in eine zwischen zwei Längswänden gebildete Vorratskammer eingebracht, die mit einem Schubförderer für das Gärgut versehen ist. Dieser Schubförderer kann ein Schubschild aufweisen, mit dessen Hilfe das Gärgut schrittweise der Förderschnecke zugefördert wird, was jedoch aufwändige Stelltriebe für das Schubschild erfordert. Einfachere Konstruktionsverhältnisse ergeben sich, wenn als Schubförderer ein Schubboden eingesetzt werden kann, der eine in eine stirnseitige Austragsöffnung der Vorratskammer mündende Förderfläche für das Gärgut bildet (EP 1 627 832 A1). Solche Schubböden sind allerdings hinsichtlich ihrer Förderleistung beschränkt. Mit der Beschickungsrate der Gäranlagen steigt aber auch der Energiebedarf für den Antrieb der Förderschnecke, insbesondere beim Einsatz von Silage mit einem langfaserigen Gutanteil überproportional.

Wird das Gärgut in Form von komprimierten Feststoffballen eines bestimmten, vorgebbaren Gewichts chargenweise der Gäranlage zugefördert (EP 1 681 344 A1), so sind die Feststoffballen zunächst zu zerkleinern, bevor sie mittels einer Förderschnecke dem Gärbehälter zugefördert werden können. Zu diesem Zweck ist eine Zerkleinerungseinrichtung mit einem nach oben offenen Behälter vorgesehen, in dem eine mit Schneid- und Reißkanten versehene Schnecke vorgesehen ist, die eine horizontale oder vertikale Achse aufweisen kann. Die mit Hilfe eines Förderbandes oben in den Behälter abgeworfenen Feststoffballen werden somit durch die Schnecke zerkleinert, bevor das zerkleinerte Gärgut durch eine mit einem Schieber verschließbare Umfangsöffnung aus dem Behälter ausgetragen werden kann. Um dem Gärgut lose Feststoffe zumischen zu können, kann zusätzlich ein Förderband vorgesehen werden, über das solche lose Feststoffe aus einem Vorratsbehälter in den Behälter zur Zerkleinerung der Feststoffballen abgeworfen werden können. Die chargenweise Beschickung der Gäranlage mit Feststoffballen setzt eine entsprechende Pressung des Gärgutes zu Feststoffballen eines bestimmten Gewichtes voraus, was eine zur Aufnahme des Gärgutes in Vorratskammern grundsätzlich unterschiedliche Vorratshaltung darstellt.

Der Erfindung liegt somit die Aufgabe zugrunde, eine Vorrichtung der eingangs geschilderten Art zur Gärgutbeschickung einer Gäranlage so auszugestalten, dass die Beschickung der Förderschnecke über einen Schubboden der Vorratskammer vorgenommen werden kann, und zwar auch für höhere Beschickungsraten der Gäranlage. Außerdem soll der Energieeinsatz für den Antrieb der Förderschnecke beschränkt bleiben.

Die Erfindung löst die gestellte Aufgabe dadurch, dass an die stirnseitige Austragsöffnung der Vorratskammer ein gegen die Austragsöffnung umfangsseitig offener Mischbehälter angeschlossen ist, der wenigstens eine von seinem Boden aufragende, mit Zerkleinerungswerkzeugen versehene Mischerschnecke besitzt, und dass der Boden des Mischbehälters in einem Höhenabstand unterhalb der Förderfläche des Schubbodens liegt.

Mit dem Vorsehen eines Mischbehälters zwischen der Vorratskammer und der Förderschnecke zum Beschicken der Gäranlage wird die vorteilhafte Voraussetzung geschaffen, das Gärgut dosiert der Förderschnecke aufgeben zu können, weil das Gärgut mit Hilfe der vom Boden des Mischbehälters aufragenden Mischerschnecke nicht nur aufgelockert, sondern auch zerkleinert wird, was insbesondere bei einem Gärgut mit einem langfaserigen Gutanteil höhere Belastungen der Förderschnecke und ihres Antriebes vermeidet. Der Förderschnecke wird ja ein Gärgut angeboten, das eine gleichmäßige Mischung aus den zerkleinerten Faseranteilen und den übrigen Bestandteilen darstellt. Die dosierte Weitergabe des Gärgutes vom Mischbehälter an die Förderschnecke bietet darüber hinaus die Möglichkeit, den Mischbehälter mit beschränkten Gärgutraten zu beschicken, was wiederum den Einsatz von Schubböden erlaubt. Die vom Schubboden über das Gärgut auf die Mischerschnecke ausgeübte Querbelastung wird dadurch berücksichtigt, dass der Boden des Mischbehälters in einem Höhenabstand unterhalb der Förderfläche des Schubbodens liegt, sodass nur ein Teil der Schubkraft des Schubbodens von der Mischerschnecke aufgenommen werden muss. Außerdem ergibt sich im Bereich der Mischerschnecke unterhalb der Förderfläche des Schubbodens ein Mischerraum, der vom Schubförderer unabhängige Misch- und Zerkleinerungsbedingungen sicherstellt.

Damit der Einfluss des Schubförderers auf die Konstruktion des Mischbehälters weitgehend unterdrückt werden kann, kann zwischen der Förderfläche des Schubbodens und einem den Öffnungsbereich des Mischbehälters gegenüber der Vorratskammer begrenzenden, vom Boden des Mischbehälters aufragenden Umfangswandabschnitt eine gegen den Begrenzungsrand dieses Umfangswandabschnittes ansteigende Rampe vorgesehen werden, über die das Gärgut mit Hilfe des Schubbodens in den Mischbehälter gefördert wird. Durch diese Rampe wird ein Teil der Schubkräfte des Schubbodens über das Gehäuse des Mischbehälters abgetragen und die Mischerschnecke zusätzlich entlastet. Außerdem ergeben sich hinsichtlich der Mischbedingungen verbesserte Verhältnisse, wenn das zu mischende und dabei zu zerkleinernde Gärgut aus einer größeren axialen Höhe in den Bereich der Mischerschnecke gelangt.

Obwohl die Konstruktion des Schubbodens unterschiedlich ausfallen kann, ergeben sich besonders günstige Konstruktionsbedingungen, wenn der Schubboden aufeinander in einer Längsführung der Förderfläche verschiebbar gelagerte, gegensinnig antreibbare Schubstangen aufweist, die in Förderrichtung abwechselnd mit auf der Förderfläche gleitend abgestützten Schubelementen verbunden sind, wobei die einander paarweise bezüglich der zugehörenden Schubstange gegenüberliegenden Schubelemente eine in Förderrichtung ansteigende Anlauffläche und eine quer zur Förderrichtung verlaufende Schubfläche für das Gärgut bilden. Durch diese Maßnahmen wird erreicht, dass während des Leerhubes der beiden Schubförderer deren Schubelemente unterhalb des Fördergutes zurückgezogen werden können, um beim anschließenden Förderhub das beim Leerhub über die Anlaufflächen in den Bereich der Schubfläche gelangende Fördergut einen Förderschritt weiterzufördern. Die gegensinnige Bewegung der beiden Schubförderer unterstützt dabei das Aufschieben des Fördergutes auf die Anlaufflächen der Schubelemente, weil beim Zurückziehen der Schubelemente des einen Schubförderers in einem Leerhub die Schubelemente des anderen Schubförderers das Fördergut in einem Förderhub mit Hilfe der Schubflächen gegen die einen Leerhub ausführenden Schubelemente vorschieben.

Wird die Vorratskammer mit dem Schubboden als transportabler Container ausgebildet, so ergeben sich für die Errichtung von erfindungsgemäßen Vorrichtungen zur Gärgutbeschickung einer Gäranlage besonders günstige Konstruktionsbedingungen, weil die Vorratskammer mit dem Schubboden als vormontierte Baueinheit angeliefert werden kann, und zwar in Abmessungen, die den gesetzlichen Vorschriften für den üblichen Straßentransport entsprechen. Vor Ort bedarf es daher lediglich des Zusammenbaus mit dem Mischbehälter, der ebenfalls eine vormontierte Baueinheit darstellen kann.

In der Zeichnung ist der Erfindungsgegenstand beispielsweise dargestellt. Es zeigen
- Fig. 1: eine erfindungsgemäße Vorrichtung zur Gärgutbeschickung einer Gäranlage in einem schematischen Längsschnitt und
- Fig. 2: die Vorrichtung nach der Fig. 1 in einer vereinfachten Draufsicht.

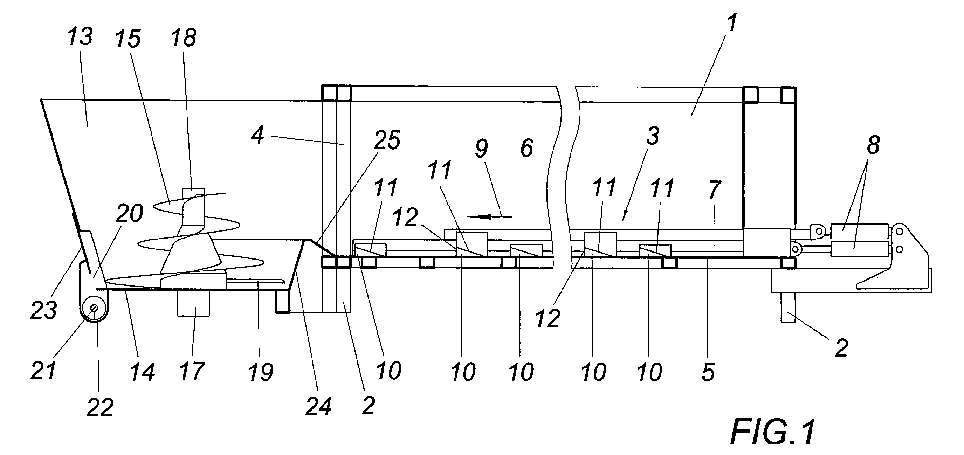

Gemäß dem dargestellten Ausführungsbeispiel ist eine Vorratskammer 1 in Form eines selbsttragenden Containers vorgesehen, der über Fußstützen 2 auf einem entsprechend vorbereiteten Untergrund aufgestellt werden kann. Diese Vorratskammer 1 weist zwei Längswände und einen zwischen diesen Längswänden vorgesehenen Schubboden 3 auf, der eine in einer stirnseitigen Austragsöffnung 4 mündende Förderfläche 5 für das Gärgut bildet. Der Schubboden 3 ist mit zwei in einer Längsführung der Förderfläche 5 aufeinander verschiebbar gelagerten Schubstangen 6, 7 versehen, die mit Hilfe von Stelltrieben 8, vorzugsweise in Form von Stellzylindern, gegensinnig angetrieben werden können. Die Schubstangen 6, 7 sind in Förderrichtung 9 abwechselnd mit auf der Förderfläche 5 gleitend abgestützten Schubelementen 10 verbunden. Die einander paarweise bezüglich der zugehörigen Schubstange 6, 7 gegenüberliegenden Schubelemente 10 bilden eine in Förderrichtung 9 ansteigende Anlauffläche 11 und eine quer zur Förderrichtung 9 verlaufende Schubfläche 12 für das Gärgut, wie dies insbesondere der Fig. 1 entnommen werden kann. Wird aus der dargestellten Endlage der Schubstangen 6, 7 die Schubstange 6 in Förderrichtung 9 in einem Förderschritt vorbewegt und zugleich die Schubstange 7 in einem Leerhub entgegen der Förderrichtung 9 zurückgezogen, so wird das in die Vorratskammer 1 eingebrachte Gärgut mit Hilfe der Schubflächen 12 der Schubelemente 10 der Schubstange 6 gegen die Anlauffläche 11 der gegensinnig bewegten Schubelemente 10 der Schubstange 7 vorgeschoben, sodass das Gärgut über die Anlauffläche 11 der sich zurückziehenden Schubelemente 10 der Schubstange 7 in den Bereich der Schubflächen 12 dieser Schubelemente gelangt, um beim nächsten Förderhub der Schubelemente 10 der Schubstange 7 in einem weiteren Förderschritt über die Anlauffläche 11 der sich dann in einem Leerhub zurückziehenden Schubelemente 10 der Schubstange 6 in den Schubbereich dieser Schubelemente gefördert zu werden, wobei im Bereich der Austragsöffnung 4 das Gärgut schrittweise aus der Vorratskammer 1 ausgefördert wird.

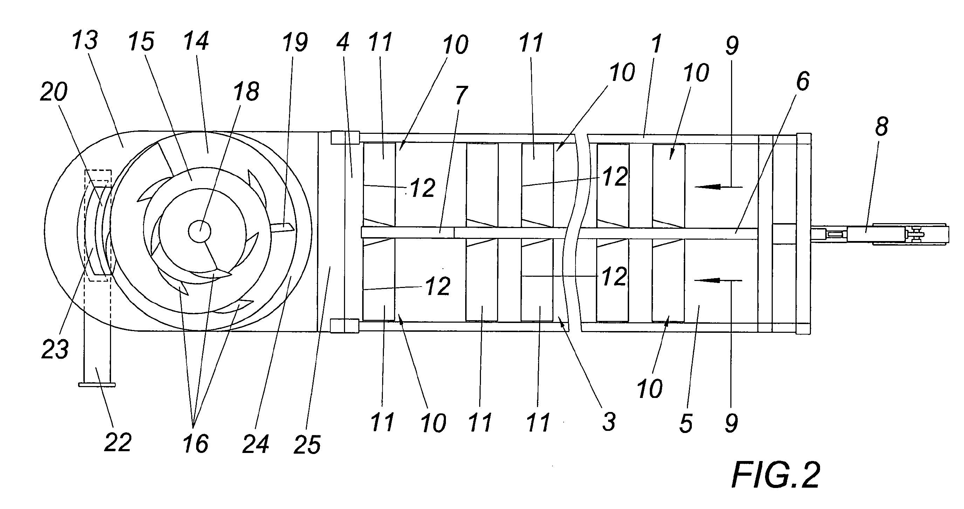

An die Austragsöffnung 4 der Vorratskammer 1 ist ein gegen diese Austragsöffnung 4 offener Mischbehälter 13 angeschlossen, der eine von seinem Boden 14 aufragende, konische Mischerschnecke 15 besitzt, die mit Zerkleinerungswerkzeugen 16 versehen ist, wie dies in der Fig. 2 angedeutet wurde. Die über einen Getriebemotor 17 angetriebene Schneckenwelle 18 ist mit wenigstens einem Räumarm 19 verbunden, der über den Boden 14 streicht und das von der Mischerschnecke 15 behandelte, zerkleinerte und gemischte Gärgut einer Austragsöffnung 20 zufördert, über die das Gärgut dosiert einer Förderschnecke 21 aufgegeben wird, deren Gehäuse mit 22 bezeichnet ist. Die Dosierung des aus dem Mischbehälter 13 ausgetragenen Gärgutes kann über einen die Öffnungsweite der Austragsöffnung 20 bestimmenden Schieber 23 eingestellt werden.

Wie der Fig. 1 entnommen werden kann, liegt der Boden 14 des Mischbehälters 13 in einem Höhenabstand unterhalb der Förderfläche 5 des Schubbodens 3. Außerdem ist zwischen der Förderfläche 5 des Schubbodens 3 und einem den Öffnungsbereich des Mischbehälters 13 gegenüber der Vorratskammer 1 begrenzenden, vom Boden 14 des Mischbehälters 13 aufragenden Umfangswandabschnitt 24 eine gegen den Begrenzungsrand dieses Umfangswandabschnittes 24 ansteigenden Rampe 25 vorgesehen, sodass das schrittweise aus der Vorratskammer 1 mit Hilfe des Schubbodens 3 ausgeförderte Gärgut über die ansteigende Rampe 25 in den Mischbereich des Mischbehälters 13 gelangt, wodurch einerseits die Mischerschnecke 15 von Querbelastungen durch die Schubkräfte des Schubbodens 3 erheblich entlastet wird und anderseits zumindest im Höhenbereich des Umfangswandabschnittes 24 vom Schubboden 3 unbeeinflußte Misch- und Zerkleinerungsbedingungen für das Gärgut geschaffen werden, sodass das Gärgut in vorteilhafter Weise aufgelockert, zerkleinert und gemischt an die Förderschnecke 21 zur Gärgutbeschickung der anschließenden Gäranlage weitergegeben werden kann.

## Patentansprüche

1. Vorrichtung zur Gärgutbeschickung einer Gäranlage mit einer das Gärgut aufnehmenden Vorratskammer (1) und mit einem in der Vorratskammer (1) vorgesehenen Schubboden (3), der eine in einer stirnseitigen Austragsöffnung (4) der Vorratskammer (1) mündende Förderfläche (5) für das Gärgut bildet, **dadurch gekennzeichnet, dass** an die stirnseitige Austragsöffnung (4) der Vorratskammer (1) ein gegen die Austragsöffnung (4) umfangsseitig offener Mischbehälter (13) angeschlossen ist, der wenigstens eine von seinem Boden (14) aufragende, mit Zerkleinerungswerkzeugen (16) versehene Mischerschnecke (15) besitzt, und dass der Boden (14) des Mischbehälters (13) in einem Höhenabstand unterhalb der Förderfläche (5) des Schubbodens (3) liegt.

2. Vorrichtung nach Anspruch 1, **dadurch gekennzeichnet, dass** zwischen der Förderfläche (5) des Schubbodens (3) und einem den Öffnungsbereich des Mischbehälters (13) gegenüber der Vorratskammer (1) begrenzenden, vom Boden (14) des Mischbehälters (13) aufragenden Umfangswandabschnitt (24) eine gegen den Begrenzungsrand dieses Umfangswandabschnittes (24) ansteigende Rampe (25) vorgesehen ist.

3. Vorrichtung nach Anspruch 1 oder 2, **dadurch gekennzeichnet, dass** der Schubboden (3) aufeinander in einer Längsführung der Förderfläche (5) verschiebbar gelagerte, gegensinnig antreibbare Schubstangen (6, 7) aufweist, die in Förderrichtung (9) abwechselnd mit auf der Förderfläche (5) gleitend abgestützten Schubelementen (10) verbunden sind, und dass die einander paarweise bezüglich der zugehörigen Schubstange (6, 7) gegenüberliegenden Schubelemente (10) eine in Förderrichtung (9) ansteigende Anlauffläche (11) und eine quer zur Förderrichtung (9) verlaufende Schubfläche (12) für das Gärgut bilden.

4. Vorrichtung nach einem der Ansprüche 1 bis 3, **dadurch gekennzeichnet, dass** die Vorratskammer (1) mit dem Schubboden (3) als transportabler Container ausgebildet ist.
